# FASCICULE DE BREVET EUROPEEN

(11) **EP 1 562 538 B1**
(45) Date de publication et mention de la délivrance du brevet: **03.11.2010**
(21) Numéro de dépôt: 03767897.6
(22) Date de dépôt: 04.11.2003
(51) Int. Cl.: A61K 8/46, A61K 8/49, A61Q 5/06

(54) **COMPOSITION COSMETIQUE COMPRENANT AU MOINS UN CARBONATE CYCLIQUE PARTICULIER APTE A POLYMERISER**
KOSMETISCHE ZUSAMMENSETZUNG ENTHALTEND MINDESTENS EIN BESTIMMTES POLYMERISIERBARES ZYKLISCHES CARBONAT
COSMETIC COMPOSITION COMPRISING AT LEAST ONE SPECIFIC CYCLIC CARBONATE WHICH MAY BE POLYMERISED

(30) Priorité: 07.11.2002 FR 0213938; 20.12.2002 US 434654 P
(43) Date de publication de la demande: 17.08.2005
(73) Titulaire: L'Oréal, 75008 Paris (FR)
(72) Inventeur: BRUN, Gaelle, F-75015 Paris (FR); ROLLAT-CORVOL, Isabelle, F-75017 Paris (FR)
(74) Mandataire: Bourdeau, Françoise
(86) Numéro de dépôt international: PCT/FR2003/003285
(87) Numéro de publication internationale: WO 2004/043330

(56) Documents cités:
- EP-A- 0 380 312
- EP-A- 0 639 369
- EP-A- 0 639 369
- EP-A- 1 022 014
- EP-A- 1 022 014
- WO-A-01/47368
- WO-A-01/47368
- WO-A-98/47995
- WO-A-99/43667
- DE-A- 19 851 451
- DE-A- 19 851 451
- US-A- 4 402 985
- US-A- 4 402 985
- PATENT ABSTRACTS OF JAPAN vol. 2000, no. 20, 10 juillet 2001 (2001-07-10) & JP 2001 072553 A (LION CORP), 21 mars 2001 (2001-03-21)
- SCHMITZ F ET AL: "ALTERNATING COPOLYMERS OF TETRAMETHYLENE UREA WITH 2,2-DIMETHYLTRIMETHYLENE CARBONATE AND ETHYLENE CARBONATE;PREPARATION OF THE CORRESPONDING POLYURETHANES" MACROMOLECULAR: RAPID COMMUNICATIONS, WILEY VCH, WEINHEIM, DE, vol. 18, no. 8, 1 août 1997 (1997-08-01), pages 699-706, XP000698408 ISSN: 1022-1336
- PATENT ABSTRACTS OF JAPAN vol. 2000, no. 20, 10 juillet 2001 (2001-07-10) & JP 2001 072553 A (LION CORP), 21 mars 2001 (2001-03-21)
- SCHMITZ F ET AL: "ALTERNATING COPOLYMERS OF TETRAMETHYLENE UREA WITH 2,2-DIMETHYLTRIMETHYLENE CARBONATE AND ETHYLENE CARBONATE;PREPARATION OF THE CORRESPONDING POLYURETHANES" MACROMOLECULAR: RAPID COMMUNICATIONS, WILEY VCH, WEINHEIM, DE, vol. 18, no. 8, 1 août 1997 (1997-08-01), pages 699-706, XP000698408 ISSN: 1022-1336

## Description

L'invention a pour objet une composition cosmétique comprenant, dans un milieu cosmétiquement acceptable, au moins un carbonate cyclique particulier en association avec au moins un composé choisi parmi les polymères fixants ou conditionneurs, les colorants directs anionique ou cationiques quaternisés pour les cheveux, les colorants d'oxydation pour les cheveux, les réducteurs, les tensioactifs et les agents oxydants. Elle vise également l'utilisation de ces carbonates cycliques en capillaire, pour le traitement des cheveux, en particulier pour donner du corps, du volume ou du gonflant aux cheveux.

Au sens de la présente invention, on entend par "*traitement du cheveu*", toute action cosmétique qui modifie les propriétés du cheveu. Ces propriétés peuvent être de nature physicochimique ou sensorielle.

Au sens de la présente invention, on entend par « *composition pour donner du corps, du volume ou du gonflant aux cheveux* », une composition qui a pour effet d'augmenter le volume global de la coiffure, en donnant aux cheveux de la vigueur et du ressort. On entend par « *composition de coiffage »,* une composition capillaire destinée au maintien et/ou à la fixation de la coiffure.

Au sens de la présente invention, on entend par "*carbonate cyclique*", un sel ou éther sel de l'acide carbonique comprenant au moins un cycle en C3 à C35.

On connaît déjà des produits cosmétiques capillaires procurant du gonflant aux cheveux. Ces produits, à base de polymères filmogènes, sont particulièrement appréciés par les utilisateurs ayant des cheveux fins.

Toutefois, une de leurs limites réside dans le fait que le gonflant disparaît dès le premier shampooing. De plus, ces produits laissent un toucher rêche, qui résulte du dépôt de polymères à la surface des cheveux.

Pour donner du gonflant aux cheveux, il est également possible d'effectuer des permanentes. Ces dernières, basées sur l'utilisation de réducteurs et d'oxydants, nécessitent une mise sous tension mécanique des cheveux, par exemple au moyen de rouleaux. Si de tels traitements ont effectivement pour effet d'augmenter le volume des cheveux, ils peuvent néanmoins présenter l'inconvénient d'altérer l'état de la fibre capillaire.

De même, s'il existe déjà des produits de coloration capables de donner du corps aux cheveux, il serait souhaitable qu'ils procurent encore un plus bel effet coiffant, plus durable et qu'ils abîment moins l'état des cheveux.

De même encore, il serait souhaitable que les shampooings procurent aux cheveux davantage de volume et de meilleures propriétés cosmétiques, tout en conservant un pouvoir lavant appréciable.

De façon générale, il serait également souhaitable de réaliser des compositions cosmétiques qui soient plus rémanentes aux shampooings que celles qui existent actuellement.

Les documents EP1 022 014, US 4 746 322, US 4 402 985 et DE 198 51 451 décrivent l'utilisation de certains carbonates cycliques en cosmétiques, servant par exemple de solvant. Néanmoins, les carbonates cycliques décrits dans ces documents ne sont pas capables de polymériser sous l'action d'une stimulation extérieure.

Certaines méthodes ont été décrites pour apporter du corps, de la masse aux cheveux, notamment aux cheveux fins, et ceci de manière rémanente. La méthode la plus couramment décrite est la polymérisation d'une molécule à l'intérieur de la fibre. Cependant, les systèmes décrits utilisent souvent des précurseurs toxiques et les conditions opératoires nécessaires à la polymérisation, par exemple une oxydation, induisent une dégradation notable de la fibre.

Le problème posé par l'invention est de trouver des compositions capillaires permettant le traitement des cheveux et qui procurent notamment aux cheveux du gonflant ou du coiffant, tout en améliorant la résistance aux shampooings, en particulier après des colorations, sans présenter l'ensemble des inconvénients énumérés ci-dessus.

Pour résoudre ce problème, l'invention propose l'utilisation cosmétique, dans un milieu cosmétiquement acceptable, d'un carbonate cyclique apte à polymériser sous l'action d'une stimulation extérieure,
le carbonate est un composé de formule (1) 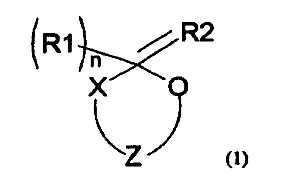 dans laquelle:
- X=O, S,
- n= 0
- R2 = O, S,
- Z désigne un radical alkylène divalent, linéaire ou ramifié, en C2 à C30, éventuellement interrompu par un ou plusieurs hétéroatomes, et éventuellement substitué, sur la chaîne principale ou sur la ramification, par un ou plusieurs radicaux aryle en C6 à C30, amino, halogène, carboxy, thiol,

Z étant différent de l'éthyléne, du propylène et de l'isopropyl-2,2'-diméthylpropylène.

Avantageusement, la stimulation extérieure est choisie parmi un amorceur de polymérisation radicalaire ou anionique, une élévation ou une baisse de température, de la lumière, et plus avantageusement, un amorceur de polymérisation anionique, tout particulièrement, de l'eau.

Selon un mode de réalisation préféré de l'utilisation selon l'invention, on choisit, comme stimulation extérieure, l'eau présente sur les cheveux, provenant notamment des cheveux mouillés ou de l'humidité de l'air.

L'utilisation selon l'invention a lieu, de préférence, pour le coiffage, le soin, le lavage, la coloration, le maquillage ou la mise en forme des cheveux, et notamment soit pour donner du corps, du volume ou du gonflant aux cheveux, soit pour améliorer la résistance aux shampooings, en particulier après des colorations.

Avantageusement, dans la formule (I),
S i X = O, R2 = O, n = 0, alors Z est différent de l'éthylène, de l'isopropylène, du propylène, du 2-hydroxypropylène, de l'isopropyl-2,2'-diméthyl propylène.

Avantageusement, dans la formule (I), Z désigne un radical alkylène divalent, linéaire ou ramifié, en C4 à C30, de préférence en C6 à C28.

Un autre objet de l'invention concerne une composition cosmétique, comprenant, dans un milieu cosmétiquement acceptable, une association formée par (i) au moins un composé choisi parmi les polymères fixants anioniques, non ioniques ou amphotères, les agents conditionneurs, les colorants directs anioniques ou cationiques quaternisés, les colorants d'oxydation pour les cheveux, les réducteurs, les tensioactifs et les agents oxydants avec (ii) au moins carbonate cyclique apte à polymériser sous l'action d'une stimulation extérieure, le carbonate cyclique est un composé de formule (1) 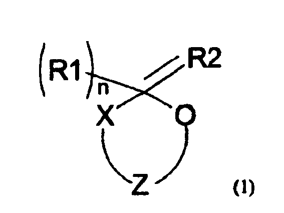 dans laquelle:
- X=O,S,
- n=0
- R2 = O, S,
- Z désigne un radical alkylène divalent, linéaire ou ramifié, en C2 à C30, éventuellement interrompu par un ou plusieurs hétéroatomes, et éventuellement substitué, sur la chaîne principale ou sur la ramification, par un ou plusieurs radicaux aryle en C6 à C30, amino, halogène, carboxy thiol,
- Z étant différent de l'éthylène, du propylène et de l'isopropyl-2,2'-diméthylpropylène.

De préférence, on choisit comme initiateur, l'octanoate d'étain, acétate ou carbonate de potassium en combinaison éventuelle avec un éther couronne, hydrure de sodium, trichlorure d'aluminium, oxyde de dibutyl étain, isopropoxyde d'aluminium ou diéthyl zinc.

### COMPOSE DE FORMULE (1)

De préférence, l'hétérocycle oxygéné contenant Z est un hétérocycle comportant au moins six chaînons.

De préférence, Z désigne un radical en C3-C15, plus préférentiellement en C3-C10, et encore plus préférentiellement en C3-C6.

On préfère les composés (1) choisis parmi ceux qui satisfont aux critères ci-après:
(i) X= O, n= 0, R2=S, Z= CH₂-CH(R)
(ii) X= O, n= 0, R2=S, Z= CH₂-CH(R)-CH₂
(iii) X= S, n= 0, R2=S, Z= CH₂-CH(R)
(iv) X= O, n= 0, R2=O, Z= CH₂-C(R, R')-CH₂
(v) X= O, n= 0, R2=O, Z= CH₂-CH₂-CH₂-CH₂

Les composés (1) visés par la présente invention sont notamment ceux décrits dans les documents ci-après:
- Nemoto, N.; Xu.X., Sanda, F.; Endo, T., Macromolecules 2001, 34,7642-7647;
- Kakimoto, K.; Nemoto, N., Sanda, F.;Endo, T.; Chemistry Letters 2002, 156-157;
- Endo et al., Macromol. Symp. 2000, 157, 21-28;
- Takata, T.; Endo, T.; Prog. Polym. Sci, 1993, 18, 839-870;
- Endo T.; Sanda F., Macromol. Symp. 2000, 159, 1-7;
- Hitomi, M.; Xu X.; Sanda, F.; Endo T.; Macromol. Chem Phys. , 1999, 200, 1268-1273_{;}
- Takata, T et al., Prog. Polym. Sci., 1992, 18, 839-870,
- Anda et al., Macromol. Rapid Commun, 1997, 18, 461-469,
- Matsuo et al., Macromol. Chem. Phys., 1998, 199, 97-102,
- Kihira et al., T. Poly(hydroxyurethane), 1993, 2765-2773.

Les documents précités indiquent également différents procédés de préparation des carbonates cycliques selon l'invention.

De préférence, dans la composition, la concentration en composé (1) est comprise entre 0,001 et 50 %, de préférence entre 0,01 et 30 %, et plus préférentiellement encore entre 0,1 et 20 % en poids par rapport au poids total de la composition.

Sans être liée par la théorie, la Demanderesse pense que l'application de la composition conforme à l'invention amène à la formation dans, à la surface ou entre les cheveux d'un matériau polymérique ou oligomérique, linéaire, branché, ramifié ou non.

Le matériau ainsi formé peut renforcer, faire gonfler le cheveu, et donc donner aux cheveux un effet de texture, de renforcement, de masse ou de volume. Si la polymérisation ayant eu lieu à l'intérieur de la fibre, le polymère formé est ensuite trop volumineux pour en ressortir ce qui assure une rémanence de ces propriétés aux shampooings.

Les propriétés suivantes peuvent aussi être changées :
- la vitesse de séchage,
- la reprise d'eau
- le comportement en milieu humide, pour une meilleure résistance de la mise en plis,
- le comportement en cas de coloration (protection des zones sensibilisées, meilleure montée et résistance de la couleur).

Lorsque cette composition est ajoutée à celles de colorants, la résistance de la couleur peut être aussi améliorée.

Il est aussi possible de modifier le toucher des cheveux, donner un effet coiffant, modifier les propriétés de démêlage et de séchage.

Les composés de l'invention peuvent aussi permettre des soudures entre les cheveux et un effet de maintien de la coiffure est obtenu. Cet effet peut résister aux shampooings.

Le matériau formé peut être dépolymérisé. Un intérêt particulier tient au fait qu'il est possible de modifier ou dégrader les propriétés physiques (solidité, insolubilité, tension de surface) du matériau après qu'il ait été formé. Il est, en effet, possible de dégrader la solidité ou l'insolubilité du matériau par dépolymérisation (réaction inverse amenant à réduire la taille du polymère, voire à revenir à l'état de monomère (le monomère pouvant alors être dans un état où le cycle initial est ouvert)) et ce par emploi de chaleur. Cette propriété est d'autant plus utile que les matériaux peuvent être rémanents aux shampooings. Cette modification peut être réalisée après application du procédé (si l'on ne souhaite pas garder l'effet obtenu) ou nettement plus tard (quelques heures ou quelques jours ou plus tard éventuellement).

### TENSIOACTIF

Selon l'invention, les agents tensioactifs peuvent être des agents tensioactifs non ioniques, cationiques, anioniques ou amphotères.

A titre d'exemples d'agents tensioactifs anioniques, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkyl-sulfates, les alkyléther-sulfates, les alkylamidoéthersulfates, les alkylarylpolyéthersulfates, les monoglycéride-sulfates, les alkylsulfonates, les phosphates d'alkyle, les alkylamidesulfonates, les alkylarylsulfonates, les α-oléfine-sulfonates, les paraffine-sulfonates, les sulfosuccinates d'alkyle, les alkyléthersulfosuccinates, les alkylamidesulfosuccinates, les sulfoacétates d'alkyle, les acylsarcosinates, et les acylglutamates, les groupes alkyle ou acyle de tous ces composés comportant de 6 à 24 atomes de carbone et le groupe aryle désignant de préférence un groupe phényle ou benzyle.

On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras, les amides gras polyéthoxylés ayant de préférence de 2 à 30 moles d'oxyde d'éthylène, les amides gras polyglycérolés comportant en moyenne de 1 à 5 et en particulier de 1,5 à 4 groupements glycérol, les esters d'acides gras du sorbitane éthoxylés ayant de 2 à 30 moles d'oxyde d'éthylène, les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine, ainsi que des mélanges de ceux-ci.

Les agents tensioactifs amphotères peuvent être notamment des dérivés d'amines aliphatiques secondaires ou tertiaires dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes, et leurs mélanges.

Parmi les dérivés d'amines, on peut citer les produits commercialisés sous la dénomination MIRANOL^{®}, tels que ceux décrits dans les brevets US 2,528,378 et US 2,781,354 et classés dans le dictionnaire CTFA, 3ème édition, 1982, sous les dénominations Amphocarboxyglycinate et Amphocarboxypropionate correspondant respectivement aux formules (a) et (b) :

R₂-CONHCH₂CH₂-N⁺(R₃)(R₄)(CH₂COO⁻) (a)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R₂'-CONHCH₂CH₂-N(B)(C) (b)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂' représente un groupe alkyle d'un acide R₂'-COOH présent dans l'huile de coprah ou dans l'huile de lin hydrolysée, un groupe alkyle, notamment en C₁₇ et sa forme iso, ou un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipmpionique, acide cocoamphodipropionique.

A titre d'exemple, on peut citer le cocoamphodiacétate commercialisé sous la dénomination commerciale MIRANOL^{®} C2M concentré par la société RHODIA.

La composition selon l'invention peut comprendre un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux qui présentent la formule générale (VI) suivante : 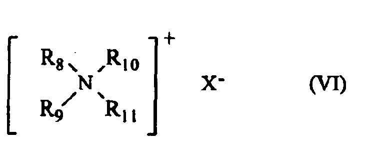 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (VII) suivante : 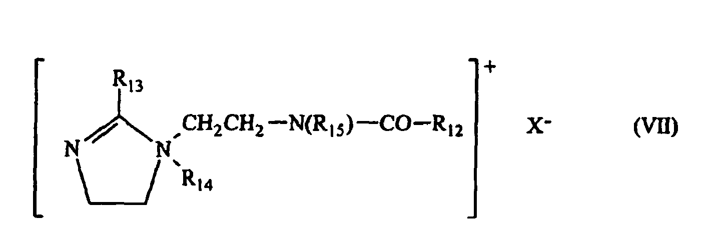 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO;
- les sels de diammonium quaternaire de formule (VIII) : 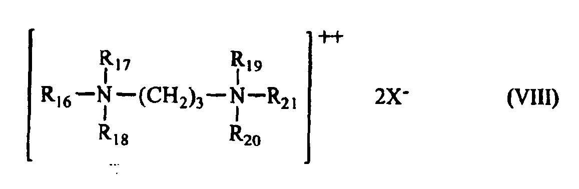 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IX) suivante : 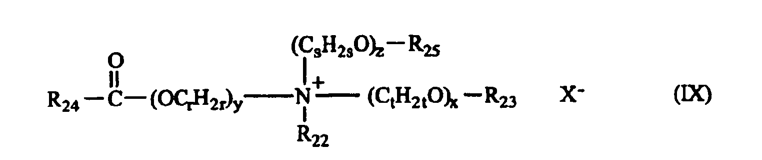
dans laquelle :
R₂₂ est choisi parmi les radicaux alkyles en C₁-C₆ et les radicaux hydroxyalkyles ou dihydroxyalkyles en C₁-C₆;
R₂₃ est choisi parmi :
   - le radical 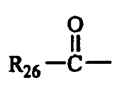
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 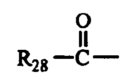
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IX) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 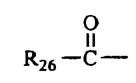
   - les radicaux méthyle, éthyle ou hydrocarbonés en C₁₄-C₂₂,
   - l'atome d'hydrogène;
- R₂₅ est choisi parmi :
   - le radical 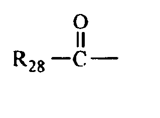
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IX) tels que les sels (chlorure ou méthylsulfate notamment) de diacyloxyéthyl-diméthylammonium, de diacyloxyéthyl-hydmxyéthyl-méthylammonium, de monoacyloxyéthyl-dihydroxyéthylméthylammonium, de triacyloxyéthyl-méthylammonium, de monoacyloxyéthylhydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyidiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention peut contenir un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyldihydroxyéthyl-méthylammonium, 45 à 60% de méthylsulfate de diacyloxyéthylhydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium et le chlorure de palmitylamidopropyltriméthylammonium.

### POLYMERE FIXANT ANIONIQUE OU NON IONIQUE OU AMPHOTERE

Au sens de la présente invention, on entend par "*polymère fixant*", un polymère capable de maintenir et/ou de fixer la coiffure dans une forme désirée.

Les polymères fixants anioniques généralement utilisés sont des polymères comportant des groupements dérivés d'acide carboxylique, d'acide sulfonique ou d'acide phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 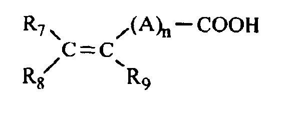 dans laquelle
n est un nombre entier de 0 à 10,
A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre,
R₇ désigne un atome d'hydrogène, un groupement phényle ou benzyle,
R₈ désigne un atome d'hydrogène, un groupement alkyle inférieur ou carboxyle, et
R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymèrs d'acide acrylique et d'acrylamide vendus sous la forme de sel de sodium sous les dénominations RETEN^{®} 421, 423 ou 425 par la société HERCULES, et les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glyeol tel que le polyéthylène-glycol, et éventuellement réticulés. De tels polymères sont décrits en particulier dans le brevet français FR 1 222 944 et la demande de brevet allemand DE 2 330 956, les copolymères de ce type comportant dans leur chaîne un motif acrylamide éventuellement N-alkylé et/ou N-hydroxyalkylé tels que décrits notamment dans les demandes de brevets luxembourgeois n° 75370 et 75371 et proposés sous la dénomination QUADRAMER^{®} par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisée par la société NATIONAL STARCH.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n° 2,047,398, 2,723,248 et 2,102,113, et le brevet GB 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

Ces polymères sont par exemple décrits dans les brevets français FR 2 350 384 et FR 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrèno-sulfonique tels que les sels de sodium vendus par exemple sous les dénominations FLEXAN^{®} 130 et FLEXAN^{®} 500 par la société NATIONAL STARCH. Ces composés sont décrits dans le brevet FR 2 198 719.
- les sels d'acides polyacrylamide-sulfoniques tels que ceux mentionnés dans le brevet US 4,128,631, et plus particulièrement l'acide polyacrylamidoéthylpropanesulfonique vendu sous la dénomination COSMEDIA POLYMER^{®} HSP 1180 par la société HENKEL.

Les polymères fixants amphotères utilisables dans les compositions de la présente invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère comportant un ou plusieurs groupements carboxyliques ou sulfoniques. Les polymères fixants amphotères peuvent également comporter des motifs zwitterioniques de type carboxybétaïne ou sulfobétaïne.

Il peut également s'agir de polymères à chaîne principale cationique comportant des groupements amine primaire, secondaire, tertiaire ou quaternaire, parmi lesquels au moins un porte, par l'intermédiaire d'un radical hydrocarboné, un groupement acide carboxylique ou acide sulfonique. Les polymères fixants amphotères peuvent encore avoir une chaîne anionique dérivée d'acides carboxyliques a,b-insaurés don l'un des groupements carboxyle a été amené à réagir avec une polyamine comportant un ou plusieurs groupements amine primaire.

Les polymères fixants amphotères répondant à la définition donnée ci-dessus sont choisis notamment parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloroacrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain US 3,836,537.
(2) les polymères comportant des motifs dérivant :
   (a) d'au moins un monomère choisi parmi les acrylamides ou les méthacrylamides substitués sur l'atome d'azote par un groupe alkyle,
   (b) d'au moins un comonomère acide contenant un ou plusieurs groupements carboxyliques réactifs, et
   (c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués sont notamment les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, furnarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et alcoylés partiellement ou totalement dérivant de polyaminoamides de formule générale :

   -(CO-R₁₀-CO-Z-)-

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe

      -NH-[(CH₂)ₓ-NH]ₚ-

      où x = 2 et p = 2 ou 3, ou bien x = 3 et p = 2
      ce groupe dérivant de la diéthylène-triamine, de la triéthylène-tétraamine ou de la dipropylène-triamine;
   b) dans les proportions de 0 à 40 % en moles, le groupe

      -NH-[(CH₂)ₓ-NH]ₚ-

      où x = 2 et p = 1 et qui dérive de l'éthylène-diamine, ou le groupe dérivant de la pipérazine : 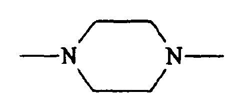
   c) dans les proportions de 0 à 20 % en moles, le groupe

      -NH-(CH₂)₆-NH-
   dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et alcoylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'alcoylation sont de préférence la propane- ou la butane-sultone, les sels des agents d'alcoylation sont de préférence les sels de sodium ou de potassium.
(4) les polymères comportant des motifs zwittérioniques de formule : 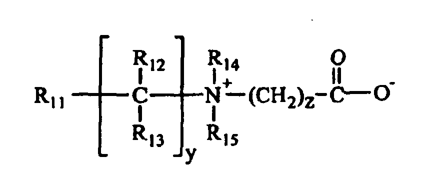 dans laquelle R₁₁ désigne un groupement insaturé polymérisable tel qu'un groupement acrylate, méthacrylate, acrylamide ou méthacrylamide, y et z représentent chacun un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthylcarboxyméthylammonioéthylméthacrylate de méthyle, tels que le produit vendu sous la dénomination DIAFORMER^{®} Z301 par la société SANDOZ.
(5) les polymères dérivés du chitosane comportant des motifs monomères répondant aux formules suivantes : 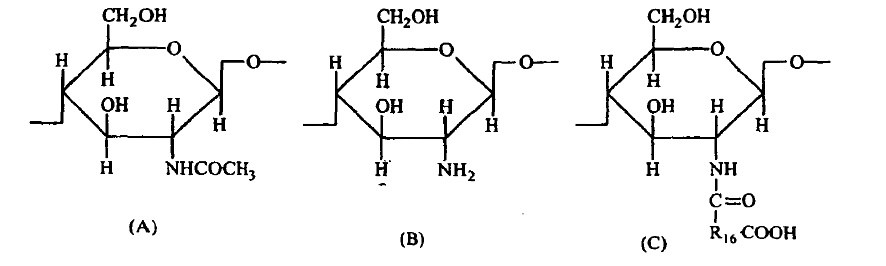 le motif (A) étant présent dans des proportions comprises entre 0 et 30%, le motif (B) dans des proportions comprises entre 5 et 50% et le motif (C) dans des proportions comprises entre 30 et 90%, étant entendu que dans ce motif (C), R₁₆ représente un groupe de formule : 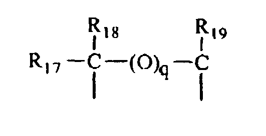 dans laquelle si q = 0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q = 1, R₁₇, R₁₈ et R₁₉ représentent chacun un atome d'hydrogène, ainsi que les sels formés par ces composés avec des bases ou des acides.
(6) Les polymères obtenus par N-carboxylation du chitosane tels que le N-carboxyméthylchitosane ou le N-carboxybutylchitosane vendu sous la dénomination EVALSAN^{®} par la société JAN DEKKER.
(7) Les polymères décrits dans le brevet français FR 1400 366 et répondant à la formule 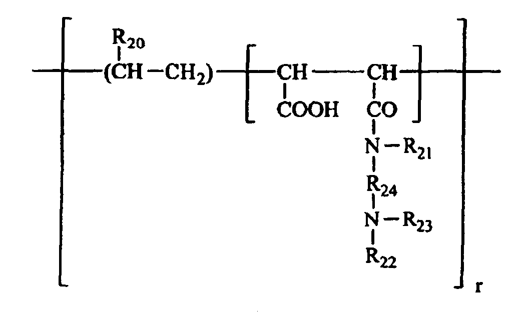 dans laquelle R₂₀ représente un atome d'hydrogène ou un groupe CH₃O, CH₃CH₂O ou phényle, R₂₁ désigne un atome d'hydrogène ou un groupe alkyle inférieur tel que méthyle et éthyle, R₂₂ désigne un atome d'hydrogène ou un groupe alkyle inférieur en C₁-C₆ tel que méthyle et éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, et R₂₂ ayant les significations mentionnées ci-dessus.
(8) Les polymères amphotères du type -D-X-D-X choisis parmi:
   (a) les polymères obtenus par action de l'acide chloroacétique ou le chloroacétate de sodium sur les composés comportant au moins un motif de formule :

      -D-X-D-X-D-

      où D désigne un groupe 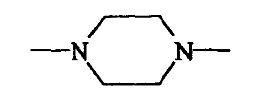 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'aminé, ammonium quaternaire, amide, imide, alcool, ester et/ou uréthanne.
   (b) Les polymères de formule :

      -D-X-D-X-

      où D désigne un groupe 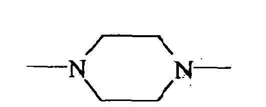 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloroacétate de soude.
(9) les copolymères alkyl(C₁-C₅)vinyléther/anhydride maléique modifiés partiellement par semi-amidification avec une N,N-dialkylammoalkylamine telle que la N,N-diméthyaminopropylamine ou par semi-estérification avec une N,N-dialcanolamine. Ces copolymères peuvent également comporter d'autres comonomères vinyliques tels que le vinylcaprolactame.

Les polymères fixants non ioniques sont choisis, par exemple, parmi :
- la vinylpyrrolidone,
- les copolymères de vinylpyrrolidone et d'acétate de vinyle,
- les polyalkyloxazolines telles que les polyéthyloxazolines proposées par la société DOW CHEMICAL sous les dénominations PEOX^{®} 50 000, PEOX^{®} 200 000 et PEOX^{®} 500 000,
- les homopolymères d'acétate de vinyle tels que le produit proposé sous la dénomination APPRETAN^{®} EM par la société HOECHST ou le produit proposé sous le nom de RHODOPAS^{®} A 012 par la société RHONE POULENC,
- les copolymères d'acétate de vinyle et d'ester acrylique tels que le produit proposé sous le nom de RHODOPAS^{®} AD 310 de la société RHONE POULENC,
- les copolymères d'acétate de vinyle et d'éthylène tels que le produit proposé sous le nom de APPRETAN^{®} TV par la société HOECHST,
- les copolymères d'acétate de vinyle et d'ester maléique, par exemple, de maléate de dibutyle tels que le produit proposé sous le nom de APPRETAN^{®} MB EXTRA par la société HOECHST,
- les copolymères de polyéthylène et d'anhydride maléique,
- les poly(acrylate d'alkyle) et poly(méthacrylate d'alkyle) tels que le produit proposé sous la dénomination MICROPEARL^{®} RQ 750 par la société MATSUMOTO ou le produit proposé sous la dénomination LUHYDRAN^{®} A 848 S par la société BASF,
- les copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAN^{®} N9212 et N9213 ;
- les copolymères d'acrylonitrile et d'un monomère non ionique choisis, par exemple, parmi le butadiène et les (méth)acrylates d'alkyle ; on peut citer les produits proposés sous les dénominations NIPOL^{®} LX 531 B par la société NIPPON ZEON ou ceux proposés sous la dénomination CJ 0601 B par la société ROHM & HAAS,
- les polyuréthannes tels que les produits proposés sous les dénominations ACRYSOL^{®} RM 1020 ou ACRYSOL^{®} RM 2020 par la société ROHM & HAAS, les produits URAFLEX^{®} XP 401 et URAFLEX^{®} XP 402 UZ par la société DSM RESINS,
- les polyamides tels que le produit ESTAPOR^{®} LO 11 proposé par la société RHONE POULENC,
- les gommes de guar non ioniques chimiquement modifiées ou non modifiées. Les gommes de guar non modifiées sont par exemple les produits vendus sous la dénomination VIDOGUM^{®} GH 175 par la société UNIPECTINE et sous la dénomination JAGUAR^{®} C par la société MEYHALL. Les gommes de guar modifiées sont de préférence modifiées par des groupements hydroxyalkyle en C₁₋₆, de préférénce par des groupements hydroxyméthyle, hydroxyéthyle, hydroxypropyle et hydroxybutyle. De telles gommes de guar non ioniques éventuellement modifiées par des groupes hydroxyalkyle sont par exemple vendues sous les dénominations commerciales JAGUAR^{®} HP8, JAGUAR^{®} HP60, JAGUAR^{®} HP120, JAGUAR^{®} DC 293 et JAGUAR^{®} HP 105 par la société MEYHALL, ou sous la dénomination GALACTOSOL^{®} 4H4FD2 par la société AQUILON.

On peut également utiliser, en tant que polymères fixants, des polymères filmogènes de type silicone greffée comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.

Ces polymères sont de préférence anioniques ou non ioniques.

De tels polymères sont par exemple les copolymères susceptibles d'être obtenus par polymérisation radicalaire à partir du mélange de monomères formé
a) de 50 à 90 % en poids d'acrylate de tertiobutyle,
b) de 0 à 40 % en poids d'acide acrylique,
c) de 5 à 40 % en poids d'un macromère siliconé de formule 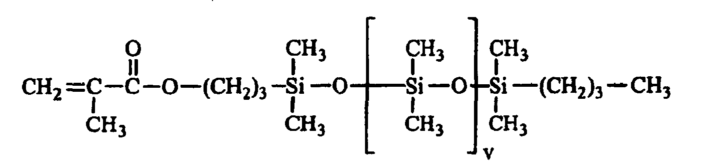
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut utiliser aussi, comme polymères fixants dans les compositions coiffantes de la présente invention des polyuréthannes fonctionnalisés ou non, siliconés ou non.

Les polyuréthannes particulièrement visés par la présente invention sont ceux décrits dans les documents EP-A-0 751 162, EP-A-0 637 600, FR 2 743 297 et EP 0 648 485 dont la demanderesse est titulaire, ainsi que les demandes EP-A-0 656 021 ou WO 94/03510 de la société BASF et la demande EP-A-0 619 111 de la société NATIONAL STARCH.

### AGENT DE CONDITIONNEMENT

Au sens de la présente invention, on entend par "*agent de conditionnement*", un composé capable d'apporter aux cheveux des propriétés cosmétiques, telles que de la douceur au toucher, du lissage ou du démêlage.

Avantageusement, l'agent de conditionnement est choisi dans le groupe comprenant les organosiloxanes, les hydrocarbures linéaires ou ramifiés en C₈ à C₃₀₀, les alcools gras linéaires ou ramifiés en C₈ à C₃₀, les esters d'acide gras en C₈ à C₃₀ et d'alcool en C₁ à C₃₀, les esters d'acide ou de diacide en C₁ à C₇ et d'alcool gras en C₈ à C₃₀, les céramides ou analogues de céramides, les polymères cationiques.

Parmi les organosiloxanes utilisés conformément à la présente invention, on peut citer à titre non limitatif :

### I. Les silicones volatiles

Celles-ci possèdent un point d'ébullition compris entre 60°C et 260°C. Parmi ce type de silicones, on cite :
(i) les silicones cycliques de 3 à 7 atomes de silicium et de préférence 4 à 5. Il s'agit, par exemple, de l'octaméthylcyclo-tétrasiloxane vendu sous le nom de "VOLATILE SILICONE 7207®" par la Société UNION CARBIDE ou "SILBIONE 70045 V2®" par la Société PHONE POULENC, le décaméthylcyclopentasiloxane vendu sous le nom de "VOLATILE SILICONE 7158®" par la Société UNION CARBIDE, "SILBIONE 70045 V5®" par la Société RHONE POULENC, ainsi que leurs mélanges.
   On cite également les cyclopolymères du type diméthylsiloxane/méthylalkylsiloxane, tel que la "SILICONE VOLATILE FZ 3109®" vendue par la Société UNION CARBIDE, qui est un cyclocopolymère diméthylsiloxane/méthyloctylsiloxane ;
(ii) les silicones volatiles linéaires ayant 2 à 9 atomes de silicium et possédant une viscosité inférieure ou égale à 5.10⁻⁶ m²/s à 25°C. Il s'agit, par exemple, de l'hexaméthyldisiloxane vendu sous la dénomination "SILBIONE 70041 V0,65®" par la Société RHONE POULENC. Ce type de produit est décrit dans l'article de TODD & BYERS "Volatile silicone fluides for cosmetics", Cosmetics and Toiletries, Vol. 91, Jan 76, pages 27-32.

### II. Les silicones non volatiles

Elles sont constituées principalement par les polyalkyl (C1-C35)-siloxanes, les polyarylsiloxanes, les polyalkyl (C1-C35)arylsiloxanes, les gommes et résines de silicone et les polysiloxanes organomodifiés, ainsi que leurs mélanges.

Parmi les polyalkylsiloxanes, on peut citer principalement les polydiméthylsiloxanes linéaires de viscosité supérieure à 5.10⁻⁶ m²/s, et de préférence inférieure à 2,6 m²/s soit :
- à groupements terminaux triméthylsilyle, comme par exemple, et à titre non limitatif, les huiles "SILBIONE®" de la série 70047 commercialisées par la Société PHONE POULENC, l'huile "47 V 500.000®" de RHONE POULENC ou certaines "VISCASIL®" de la Société GENERAL ELECTRIC,
- à groupements terminaux trihydroxysilyle, tels que les huiles de la série "48 V®" de la Société PHONE POULENC.

Dans cette classe de polyalkylsiloxanes, on peut également mentionner les polyalkylsiloxanes vendus par la Société GOLDSCHMIDT sous les dénominations "ABILWAX 9800®" et "AHILWAX 9801®", qui sont des polyalkyl(C₁-C₂₀)siloxanes.

Parmi les polyalkylarylsiloxanes, on peut citer les polydiméthylphénylsiloxanes, les polydiméthyldiphénylsiloxanes linéaires et/ou ramifiés, de viscosité 10⁻⁵ à 5.10⁻² m²/s, tels que par exemple:
- l'huile "RHODORSIL®" 763 de RHONE POULENC,
- les huiles "SILBIONE®" de la série 70641 de RHONE POULENC, telles que les huiles "SILBIONE 70641 V30®" et "SILBIONE 70641 V200®" de PHONE POULENC,
- le produit "DC 556®" Cosmetic Grad Fluid de Dow CORNING,
- les silicones de séries PK de BAYER, telles que la "PK20®",
- les silicones des séries PN, PH de BAYER, comme les "PN 1000®" et "PH 1000®",
- certaines huiles des séries SF de GENERAL ELECTRIC, telles que les "SF 1250®", "SF 1265®", "SF 1154®", "SF 1023®".

Les gommes de silicone, conformes à la présente invention, sont des polydiorganosiloxanes de forte masse moléculaire moyenne en nombre comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les composés ayant les structures suivantes:
- poly[(diméthylsitoxane)/(méthylvinylsitoxane)],
- poly[(diméthylsiloxane)/(diphénylsiloxane)],
- poly[(diméthylsiloxane)/(phénylméthylsiloxane)],
- poly[(diméthylsiloxane)/(diphétlylsiloxane)/ (méthylvinylsiloxane)].

On peut citer, par exemple, à titre non limitatif, les mélanges suivants :
1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401®" vendu par la Société Dow CORNING ;
2) les mélanges formés à partir d'une gomme polydiméthylsiloxane avec une silicone cyclique, tel que le produit "SF 1214 SILICONE FLUID®" de GENERAL ELECTRIC, qui est une gomme "SE 30®" de PM 500.000 (⁻,Mn) solubilisée dans la "SF 1202 SILICONE FLUID®" (décaméthylcyclopentasiloxane) ;
3) les mélanges de deux PDMS de viscosités différentes, notamment d'une gomme PDMS et d'une huile PDMS, tels que les produits "SF 1236®" et "CF 1241®" de la Société GENERAL ELECTRIC. Le produit "SF 1236®" est le mélange d'une gomme "SE 30®" définie ci-dessus d'une viscosité de 20 m²/s et d'une huile "SF 96®" d'une viscosité de 5.10⁻⁶ m²/s (15 % de gomme "SE 30®" et 85 % d'huile "SF 96®").

Le produit "CF 1241®" est le mélange d'une gomme "SE 30®" (33 %) et d'une PDMS (67 %) de viscosité 10⁻³ m²/s.

Les résines d'organopolysiloxanes utilisables conformément à l'invention, sont des systèmes siloxaniques réticulés renfermant les unités : R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination "DOW CORNING 593®" ou ceux vendus sous les dénominations "SILICONE FLUID SS 4230" et "SILICONE FLUID SS 4267" par la Société GENERAL ELECTRIC et qui sont des diméthyl/triméthylpolysiloxanes.

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :
a) des groupements perfluorés tels que des trifluoroalkyles comme, par exemple, celles vendues par la Société GENERAL ELECTRIC sous les dénominations "FF.150 FLUOROSILICONE FLUID®" ou par la Société SHIN ETSU sous les dénominations "X-22-819®", "X-22-82®", "X-22-821®" et "X-22-822®";
b) des groupements hydroxyacylamino comme, par exemple, celles décrites dans 1a demande de brevet EP-A-0 342 834 et en particulier la silicone vendue par la Société DOW CORNING sous la dénomination "Q2-8413®" ;
c) des groupements thiols comme dans les silicones "X 2-8360®" de la Société Dow CORNING ou les "GP 72A®" et "GP 71®" de GENESEE ;
d) des groupements aminés substitués ou non, comme dans la "GP 4 SILICONE FLUID®" de GENESEE, la "GP 7100®" de GENESEE, la "Q2 8220®" de DOW CORNING, l"'AFL 40®" d'UNION CARBIDE ou la silicone dénommée "Amodiméthicone" dans le dictionnaire CTFA ;
e) les groupements carboxylates, comme les produits décrits dans le brevet EP 186 507 de CHISSO CORPORATION ;
f) des groupements hydroxylés, comme les polyorgano-siloxanes à fonction hydroxyalkyle, décrits dans la demande de brevet FR-85 16334, répondant à la formule suivante : 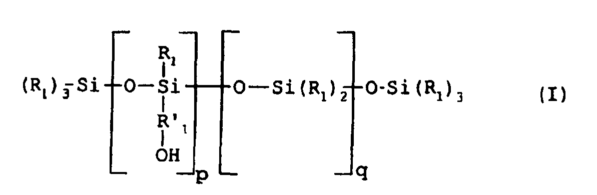 dans laquelle :
   - les radicaux R₁, identiques ou différents, sont choisis parmi les radicaux méthyle et phényle, au moins 60 % en mole des radicaux R₁ étant méthyle ;
   - le radical R'₁ est un chaînon alkylène divalent hydrocarboné en C₂-C₁₈;
   - p est compris entre 1 et 30 inclus ;
   - q est compris entre 1 et 150 inclus .
   On peut citer tout particulièrement le produit commercialisé par Dow Coming sous l'appellation DC 190 ;
g) des groupements alcoxylés comme dans la Silicone copolymer "F 755®" de SWS SILICONES et les produits "ABILWAX 2428®)", "ABILWAX 2434®", "ABILWAX 2440®" de la Société GOLDSCHMIDT ;
h) des groupements acyloxyalkyles, comme par exemple les polyorganopolysiloxanes décrits dans la demande de brevet FR-88 17433, répondant à la formule suivante : 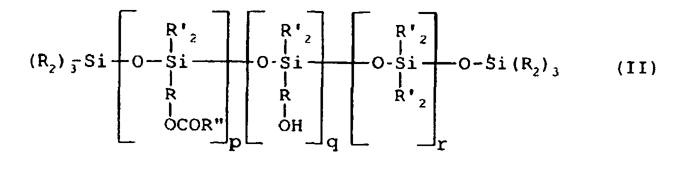 dans laquelle :
   - R₂ désigne méthyle, phényle, OCOR", hydroxyle, un seul des R₂ par atome de silicium peut être OH ;
   - R'₂ désigne méthyle, phényle, 60 % molaire au moins de l'ensemble des radicaux R₂ et R'₂ est méthyle ;
   - R" désigne alcoyle ou alcényle en C₈-C₂₀ ;
   - R désigne un alkylène hydrocarboné divalent, linéaire ou ramifié en C₂-C₁₈ ;
   - r est compris entre 1 et 120 inclus ;
   - p est compris entre 1 et 30 inclus ;
   - q vaut 0 ou est inférieur à 0,5 p, p + q étant compris entre 1 et 30 inclus :
   les polyorganosiloxanes de formule (II) peuvent contenir des groupements 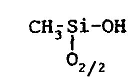 dans des proportions ne dépassant pas 15 % de la somme p + q + r ;
i) des groupements ammonium quaternaires, comme dans les produits "X2 81 08®" et "X2 81 09®", le produit "ABIL K 3270®" de la Société GOLDSCHMIDT ;
j) des groupements amphotères ou bétaïniques, tels que dans le produit vendu par la Société GOLDSCHMIDT sous la dénomination "ABIL B 9950®" ;
k) des groupements bisulfites, tels que dans les produits vendus par la Société GOLDSCHMIDT sous les dénominations "ABIL S 201®" et "ABIL S 255®" .

Les viscosités des silicones peuvent être notamment déterminées par la norme ASTM D445-97 (viscométrie).

Lorsque l'agent de conditionnement de la composition selon l'invention est un hydrocarbure, celui-ci est un hydrocarbure, linéaire ou ramifié en C₈-C₃₀₀. Parmi les hydrocarbures liquides à température ambiante répondant à cette définition, on peut notamment citer l'isododécane, l'isohexadécane et ses isomères (tels que le 2,2,4,4,6,6-heptaméthylnonane), l'isocicosane, l'isotétracosane, et les isomères desdits composés. On utilise de préférence selon l'invention l'isododécane ou l'un de ses isomères.

Lorsque l'agent de conditionnement est un alcool gras, celui-ci est du type linéaire ou ramifié, saturé ou insaturé, en C₈-C₃₀ et parmi ceux-ci on peut citer le butyl-2 octanol, l'octyldodécanol, l'alcool laurique, l'alcool oléique, l'alcool isocétylique, l'alcool isostéarique et l'alcool béhénique.

Lorsque l'agent de conditionnement est un ester gras, celui-ci peut être soit un ester d'un acide gras en C₈-C₃₀ et d'alcool en C₁-C₃₀ soit un ester d'un acide ou diacide en C₁-C₇ et d'un alcool gras en C₈-C₃₀. Parmi ces esters on peut citer le palmitate d'éthyle, d'isopropyle, d'éthyl-2-hexyle et de 2-octyldécyle, le myristate d'isopropyle, de butyle, de cétyle et de 2-octyldécyle, le stéarate de butyle et d'hexyle, le laurate d'hexyle et de 2-hexyldécyle, l'isononanoate d'isononyle et le malate de dioctyle.

Les céramides ou analogues, tels que les glycocéramides utilisables dans les compositions selon l'invention, sont connus en eux-mêmes et sont des molécules naturelles ou synthétiques pouvant répondre à la formule générale suivante : 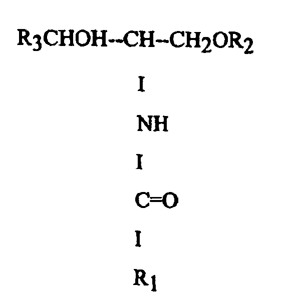 dans laquelle :
- R₁ désigne un radical alkyle, linéaire ou ramifié, saturé ou insaturé, dérivé d'acides gras en C₁₄-C₃₀, ce radical pouvant être substitué par un goupement hydroxyle en position α, ou un groupement hydroxyle en position ω estérifié par un acide gras saturé ou insaturé en C₁₆-C₃₀ ;
- R₂ désigne un atome d'hydrogène ou un radical (glycosyle)ₙ, (galactosyle)ₘ ou sulfogalactosyle, dans lesquels n est un entier variant de 1 à 4 et m est un entier variant de 1 à 8;
- R₃ désigne un radical hydrocarboné en C₁₅-C₂₆, saturé ou insaturé en position α, ce radical pouvant être substitué par un ou plusieurs radicaux alkyle en C₁-C₁₄ ;
étant entendu que dans le cas des céramides ou glycocéramides naturelles, R₃ peut également désigner un radical α-hydroxyalkyle en C₁₅-C₂₆, le groupement hydroxyle étant éventuellement estérifié par un α-hydroxyacide en C₁₆-C₃₀.

Les cétamides plus particulièrement préférés selon l'invention sont les composés pour lesquels R₁ désigne un alkyle saturé ou insaturé dérivé d'acides gras en C₁₆-C₂₂ ; R₂ désigne un atome d'hydrogène ; et R₃ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

Encore plus préférentiellement, on utilise les céramides pour lesquels R₁ désigne un radical alkyle saturé ou insaturé dérivé d'acides gras ; R₂ désigne un radical galactosyle ou sulfogalactosyle ; et R₃ désigne un groupement

-CH=CH-(CH₂)₁₂-CH₃.

Les polymères cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant des motifs correspondant à au moins une des formules suivantes : 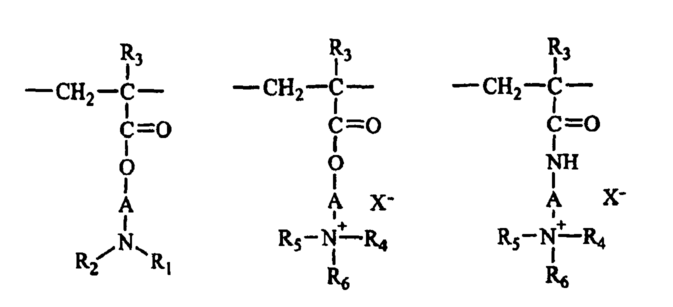 dans lesquelles:
   R₁ et R₂ représentent chacun indépendamment un atome d'hydrogène ou un groupe alkyle en C₁₋₆ ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆ représentent indépendamment chacun un groupe alkyle ayant de 1 à 18 atomes de carbone ou un groupe benzyle ;
   X désigne un anion méthosulfate ou halogénure tel que chlorure ou bromure.

   Les copolymères de la famille (1) contiennent en outre un ou plusieurs motifs dérivant de comonomères choisis dans la famille des acrylamides, méthacrylamides, diacétone-acrylamides, acrylamides et méthacrylamides substitués sur l'azote par des groupes alkyle inférieur, des acides (méth)acryliques ou de leurs esters, vinyllactames tels que la vinylpyrrolidone ou le vinylcaprolactame, esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de méthyle tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT^{®} P100 par la société CIBA GEIGY,
   - le copolymère d'acrylamide et de méthosulfate de méthacryloyloxyéthyltriméthylammonium vendu sous la dénomination RETEN^{®} vendu par la société HERCULES,
   - les copolymères vinylpyrrolidonelacrylate ou méthacrylate de dialkylaminoalkyle quaternisés ou non, tels que les produits vendus sous la dénomination GAFQUAT^{®} par la société ISP comme, par exemple, GAFQUAT^{®} 734 ou GAFQUAT^{®} 755, ou bien les produits dénommés "COPOLYMER^{®} 845, 958 et 937". Ces polymères sont décrits en détail dans les brevets français FR 2 077 143 et FR 2 393 573,
   - les terpolymères méthacrylate de diméthylaminoéthyle/ vinylcaprolactame/vinylpyrrolidone tels que le produit commercialisé sous la dénomination GAFFIX^{®} VC 713 par la société ISP, et
   - les copolymères vinylpyrrolidone/méthacrylamide de diméthylamino-propyle quaternisé tels que notamment le produit commercialisé sous la dénomination GAFQUAT^{®} HS 100 par la société ISP.
(2) les polysaccharides quaternisés décrits plus particulièrement dans les brevets américains US 3,589,578 et US 4,031,307 tels que les gommes de guar contenant des groupements cationiques trialkylammonium. De tels produits sont commercialisés notamment sous les dénominations JAGUAR C13 S, JAGUAR C 15, et JAGUAR C 17 par la société MEYHALL.
(3) les copolymères quaternaires de vinylpyrrolidone et de vinylimidazole, tels que les produits commercialisés par la société BASF sous la dénomination LUVIQUAT TFC.
(4) les chitosanes et leurs sels tels que l'acétate, le lactate, le glutamate, le gluconate ou le pyrrolidone-carboxylate de chitosane.
   Parmi ces composés, on peut citer le chitosane ayant un taux de désacétylation de 90,5 % en poids commercialisé sous la dénomination KYTAN BRUT STANDARD par la société ABER TECHNOLOGIES, le pyrrolidone-carboxylate de chitosane vendu sous la dénomination KYTAMER^{®} PC par la société AMERCHOL .
(5) les dérivés de cellulose cationiques tels que les copolymères de cellulose ou de dérivés de cellulose greffés avec un monomère hydrosoluble comportant un ammonium quaternaire, et décrits notamment dans le brevet US 4,131,576, tels que les hydroxyalkylcelluloses, comme les hydroxyméthylcelluloses, les hydroxyéthylcelluloses et hydroxypropyl-celluloses greffées notamment avec un sel de méthacryloyloxyéthyl-tri-méthylammonium, de méthacrylamidoproyltriméthylammonium ou de diméthyldiallylammonium.
   Ces polymères sont vendus notamment sous les dénominations CELQUAT^{®} L200 et CELQUAT^{®} H 100 par la société NATIONAL STARCH.
(6) Les homo- et copolymères d'halogénures de dialkyl diallylammonium, vendus notamment sous la dénomination MERQUAT ou MERQUAT 550 ou MERQUAT S par la Société Nalco.
(7) Les polycondensats à base de motifs diamines quaternisés, tels que le polyquaternium 2, vendu sous l'appellation MIRAPOLA 15 par Rhodia ou l'hexadimétrine chloride, commercialisé sous l'appellation MEXOMERE PO par Chimex.

### COLORANTS DIRECTS CATIONIQUES ET ANIONIQUES

Par "*colorant direct anionique*", on entend, au sens de la présente invention, les colorants directs présentant au moins un groupement - SO₃X ou - CO₂X dans leur structure (X= H ou métal alcalin ou alcalino-terreux ou amine organique).

Par "*colorant direct cationique quaternisés*", on entend, au sens de la présente invention, les colorants directs contenant au moins un atome d'azote quaternisé.

Parmi les colorants directs cationiques ou anioniques, on peut utiliser ceux décrits dans le COLOUR INDEX INTERNATIONAL, 3ème édition, sous les appellations BASIC dyes ou ACID dyes et ceux des demandes de brevets WO95/15144 et WO95/01772 et EP 714 954.

On peut ainsi citer parmi les colorants directs azoïques les colorants suivants, décrits dans le COLOUR INDEX INTERNATIONAL 3^{e} édition :
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24

Parmi les colorants directs synthétiques quinoniques, on peut citer les composés suivants :
- Acid Violet 43
- Acid Blue 62
- Basic Blue 22
- Basic Blue 99,
   ainsi que le composé :
- 1-N-méthylmorpholiniumpropylamino-4-hydroxyanthraquinone,

Parmi les colorants directs synthétiques aziniques, on peut citer les composés suivants :
- Basic Blue 17
- Basic Red 2.

Parmi les colorants directs synthétiques triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

### COLORANT D'OXYDATION

Les colorants d'oxydation utilisables selon l'invention sont choisis parmi les bases d'oxydation et/ou les coupleurs.

De préférence les compositions selon l'invention contiennent au moins une base d'oxydation. Les bases d'oxydation utilisables dans le cadre de la présente invention sont choisies parmi celles classiquement connues en teinture d'oxydation, et parmi lesquelles on peut notamment citer les ortho- et para-phénylènodiamines, les bases doubles, les ortho- et para- aminophénols, les bases hétérocycliques suivantes ainsi que leurs sels d'addition avec un acide.

On peut notamment citer :
- (I) les paraphénylènediamines de formule (I) suivante et leurs sels d'addition avec un acide : 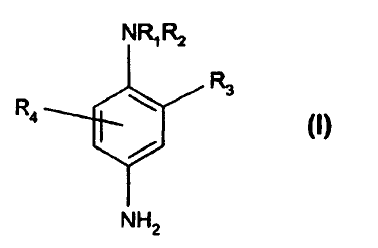 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (I) ci-dessus, on peut plus particulièrement citer la paraphénylènediamine, la paratoluylènodiamine, la 2-chloro-paraphénylèaediamine, la 2,3-diméthyl-paraphénylènediaminc, la 2,6-diméthyl-paraphénylènediarmne, la 2,6-diéthyl-paraphénylènediamine, la 2,5-diméthyl-paraphénylènediamine, la N,N-diméthyl-paraphénylènediamine, la N,N-diéthyl-pamphénylènediamine, la N,N-dipropyl-paraphénylènediamme, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthylaniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphéndiamine, la 2-hydroxyméthyl-paraphénylènediamine, la N,N-diméthyl-3-méthyl-paraphénylénediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (II) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 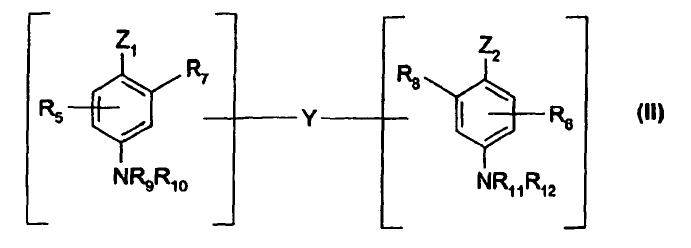 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.
   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N-bis-(β-hydroxyéthyl)-N,N'-bis-(4'-aminophényl)-1,3-diamino-propanol, la N,N'-bis-(β-hydroxyéthyl)-N,N-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (III) les para-aminophénols répondant à la formule (III) suivante, et leurs sels d'addition avec un acide : 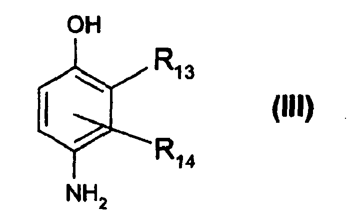 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazotiques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazole-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautemères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Les coupleurs utilisables dans le procédé de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indolc, le 4-hydmxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

### REDUCTEUR

Avantageusement, le réducteur est choisi parmi l'acide thioglycolique et ses sels, l'acide thiolactique et ses sels, la cystéine, la cystéamine et le thioglycolate de glycérol, les sulfites, les bisulfites, les sulfinates, l'acide ascorbique, l'acide érythorbique et le glucose.

### OXYDANT

Avantageusement, l'oxydant est choisi parmi le peroxyde d'hydrogène, le peroxyde d'urée, les bromates de métaux alcalins, les persels tels que les perborates et persulfates, les periodates.

De préférence, la concentration en composé choisi parmi les polymères fixants anioniques, non ioniques ou amphotères ou les agents conditionneurs, les colorants directs anioniques ou cationiques ou les colorants d'oxydation pour les cheveux, les réducteurs, les tensioactifs et les agents oxydant, est comprise entre 0,001 et 50 %, plus préférentiellement entre 0,01 et 20 %, et encore plus préférentiellement entre 0,1 et 10 %.

La composition peut contenir, en outre, par exemple, un ou plusieurs composés choisi parmi des agents de contrôle de pH, des initiateurs de polymérisation, tels que des sels métallique, des catalyseurs de polymérisation, comme des enzymes, des solvants, comme l'éthanol, des antibactériens, des nacrants, des gaz propulseurs, des particules ou nanoparticules ou des pigments.

Avantageusement, dans la composition selon l'invention, le colorant d'oxydation est une base d'oxydation et plus préférentiellement encore, elle contient au moins un coupleur.

On préfère particulièrement quand le colorant direct est choisi parmi les colorants azoïques, quinoniques, aziniques ou triarylméthaniques.

Le procédé conforme à l'invention comprend l'application de la composition sur une matière kératinique, notamment sur les cheveux. II peut s'agir, par exemple, d'une composition de coloration, de décoloration, de coiffage, de shampooing, de permanente, d'une composition avant ou après permanente, avant ou après coloration.

La composition peut, selon un mode particulier, être réalisée au moment de l'emploi. Elle peut être appliquée puis rincée, appliquée et non rincée, avec ou sans temps de pose et avec ou sans chaleur.

L'application de la composition peut être suivie ou précédée de l'application d'autres compositions. Un rinçage ou un shampooing peut être ou non intercalés entre les différentes applications.

On peut conditionner les compositions de la présente invention dans n'importe quel dispositif usuel. On pourra utiliser des dispositifs aérosols, contenant des agents propulseurs non solubles ou partiellement solubles dans la phase liquide tels que le diméthyléther, les alcanes enC₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅.

On préfère tout particulièrement utiliser comme agent propulseur les alcanes en C₃₋₅, et en particulier le propane, le n-butane et l'isobutane.

La composition peut aussi se présenter sous forme de lotions, de gels, de crèmes, de mousses ou de sticks.

La composition selon l'invention peut être appliquée, de préférence sur les cheveux, mais également sur la peau, les cils et les ongles.

L'invention concerne aussi l'utilisation cosmétique d'un carbonate de formule (1) 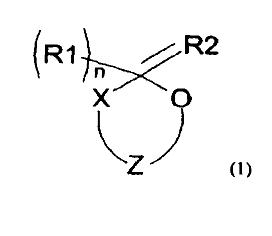 dans laquelle:
- X = O, S,
- n= 0,
- R2 = O, S,
- Z désigne un radical alkylène divalent, linéaire ou ramifié, en C4 à C30, éventuellement interrompu par un ou plusieurs hétéroatomes, et éventuellement substitué, sur la chaîne principale ou sur la ramification, par un ou plusieurs radicaux aryle en C6 à C30, amino, halogène, carboxy, thiol,

Z etant différent de l'éthylène, du propylène et de l'isopropyl-2,-2'-diméthypropylène, notamment pour donner du volume ou du gonflant aux cheveux ou pour améliorer la rémanence au shampooings de compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

L'exemple de formulation illustre la présente invention sans cependant la limiter.

### Exemple:

On applique sur une mèche de cheveux naturels de 2,7g, en utilisant la chaleur comme activateur, une composition contenant un composé de formole (I). On procède comme suit:
- Application d'une solution aqueuse contenant 10% de 5,5*'*-diméthyldioxan-2-one durant 30 minutes,
- Séchage durant une heure à l'air sans rinçage,
- Séchage durant 30 minutes à 70°C,
- Rinçage.

## Revendications

1. Utilisation cosmétique, dans un milieu cosmétiquement acceptable, d'un carbonate cyclique apte à polymériser sous l'action d'une stimulation extérieure,
le carbonate est un composé de formule (1) 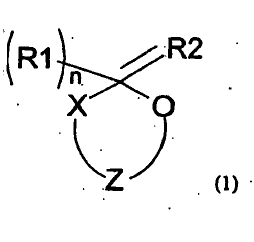 dans laquelle:
- X = O, S,
- n= 0
- R2 = O**,** S**,**
- Z désigne un radical alkylène divalent, linéaire ou ramifié, en C2 à C30, éventuellement interrompu par un ou plusieurs hétéroatomes, et éventuellement substitué, sur la chaîne principale ou sur la ramification, par un ou plusieurs radicaux aryle en C6 à C30, amino; halogène, carboxy, thiol,
Z étant différent de l'éthylène, du propylène et de l'isopropyl-2,2-diméthylpropylène.

2. Utilsation selon la revendication 1, **caractérisée par le fait que** la stimulation extérieure est choisie parmi un amorceur de polymérisation radicalaire ou anionique, une élévation ou une baisse de température, de la lumière.

3. Utilisation selon la revendication 1 ou 2, **caractérisée par le fait que** la stimulation extérieure est un amorceur de polymérisation anionique.

4. Utilisation selon la revendication 3, **caractérisée par le fait que** l'amorceur est de l'eau.

5. Utilisation selon l'une quelconques des revendications 1 à 4 en cosmétique capillaire.

6. Utilisation selon la revendication 5 pour le coiffage, le soin, le lavage, la coloration, le maquillage ou la mise en forme des cheveux.

7. Utilisation selon la revendication 5 pour donner du corps, du volume ou du gonflant aux cheveux.

8. Utilisation selon la revendication 5 pour améliorer la résistance aux shampooings, en particulier après des colorations.

9. Utilisation selon l'une quelconque des revendications précédentes, **caractérisée par le fait que** Z désigne un radical alkylène divalent, linéaire ou ramifié, en C4 à C30, de préférence en C6 à C28.

10. Utilisation selon l'une quelconque des revendications précédentes, **caractérisée par le fait que** l'hétérocycle oxygéné contenant Z est un hétérocycle comportant au moins six chaînons.

11. Utilisation selon l'une quelconque des revendications précédentes, **caractérisée par le fait que** le composé (1) est choisi parmi ceux qui satisfont aux critères ci-après:
(i) X= O, n= 0, R2=S, Z= CH₂-CH(R)
(ii) X= O, n= 0, R2=S, Z= CH₂-CH(R)-CH₂
(iii) X= S, n= 0, R2=S, Z= CH₂-CH(R)
(iv) X= O, n= 0, R2=O, Z= CH₂-C(R, R')-CH₂
(v) X= O, n= 0, R2=O, Z= CH₂-CH₂-CH₂-CH₂

12. Utilisation selon l'une quelconque des revendications précédentes, **caractérisée par le fait que** le composé (1) est mis en oeuvre, dans une composition capillaire, en association avec au moins un composé choisi parmi les polymères fixants anioniques, non ioniques ou amphotères, les agents conditionneurs, les colorants directs anioniques ou cationiques quaternisés, les colorants d'oxydation pour les cheveux, les réducteurs, les tensioactifs et les agents oxydants.

13. Composition cosmétique, comprenant, dans un milieu cosmétiquement acceptable, une association formée par (i) au moins un composé choisi parmi les polymères fixants anioniques, non ioniques ou amphotères, les agents conditionneurs, les colorants directs anioniques ou cationiques quaternisés, les colorants d'oxydation pour les cheveux, les réducteurs, les tensioactifs et les agents oxydants avec (ii) au moins un carbonate cyclique apte à polymériser sous l'action d'une stimulation extérieure, le carbonate cyclique est un composé de formule (1) 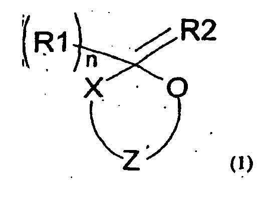 dans laquelle:
- X = O, S,
- n= 0
- R2 = O, S,
- Z désigne un radical alkylène divalent, linéaire ou ramifié, en C2 à C30, éventuellement interrompu par un ou plusieurs hétéroatomes, et éventuellement substitué, sur la chaîne principale ou sur la ramification, par un ou plusieurs radicaux aryle en C6 à C30, amino, halogène, carboxyl thiol,
Z étant différent de l'éthylène, du propylène et de l'isopropyl-2,2'-diméthylpropylène.

14. Composition selon la revendication 13, **caractérisée par le fait que** Z désigne un radical radical alkylene divalent, linéaire ou ramifié; en C4 à C30, de préférence en C6 à C28.

15. Composition selon la revendication 13 à 14, **caractérisée par le fait que** la concentration en composé (1) est comprise entre 0,001 et 50 %, de préférence entre 0,01 et 30 %, et plus préférentiellement encore entre 0,1 et 20% en poids par rapport au poids total de la composition.

16. Composition selon l'une quelconque des revendications 13 à 15, **caractérisée par le fait que** la concentration en composé choisi parmi les polymères fixants anioniques, non ioniques ou amphotères ou les agents conditionneurs, les colorants directs anioniques ou cationiques ou les colorants d'oxydation pour les cheveux, les réducteurs, les tensioactifs et les agents oxydant, est comprise entre 0,001 et 50 %, plus préférentiellement entre 0,01 et 20 %, et encore plus préférentiellerment entre 0,1 et 10 %.

17. Composition selon l'une quelconque des revendications 13 à 16, **caractérisée par le fait que** le réducteur est choisi parmi l'acide thioglycolique et ses sels, l'acide thiolactique et ses sels, la cystéine, la cystéamine et le thioglycolate de glycérol, les sulfites, les bisulfites, les sulfinates, l'acide ascorbique, l'acide érythorbique et le glucose.

18. Composition selon l'une quelconque des revendications 13 à 16, **caractérisée par le fait que** l'oxydant est choisi parmi le peroxyde d'hydrogène, le peroxyde d'urée, les bromates de métaux alcalins, les persels tels que les perborates et persulfates, les perborates.

19. Composition selon l'une quelconque des revendications13 à 16, **caractérisée par le fait que** le tensioactif est choisis parmi les tensioactifs anioniques, cationiques, non ioniques ou amphotères.

20. Composition selon l'une quelconque des revendications 13 à 16, **caractérisée par le fait que** le colorant d'oxydation est une base d'oxydation.

21. Composition selon la revendication 20, **caractérisée par le fait qu'**elle contient au moins un coupleur.

22. Composition selon l'une quelconque des revendications 13 à 16, **caractérisée par le fait que** le colorant direct est choisi parmi les colorants azoïques, quinoniques, aziniques ou triarylméthaniques.

23. Composition selon l'une quelconque des revendications 13 à 22, **caractérisée par le fait qu'**elle contient, en outre, un ou plusieurs composés choisis parmi des agents de contrôle de pH, des initiateurs de polymérisation, tels que des sels métalliques, des catalyseurs de polymérisation, comme des enzymes, des solvants, comme l'éthanol, des antibactériens, des nacrants, des gaz propulseurs, des particules ou nanoparticules ou des pigments.

## Claims

1. Cosmetic use, in a cosmetically acceptable medium, of a cyclic carbonate capable of polymerizing under the action of external stimulation, the carbonate being a compound of formula (1) 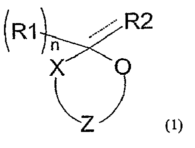 in which:
- X = O, S,
- n = 0,
- R2 = O, S,
- Z denotes a linear or branched C2 to C30 divalent alkylene radical, optionally interrupted with one or more heteroatoms, and optionally substituted, on the main chain or on the branching, with one or more C6 to C30 aryl, amino, halogen, carboxyl or thiol radicals,
Z being other than ethylene, propylene and isopropyl-2,2'-dimethylpropylene.

2. Use according to Claim 1, **characterized in that** the external stimulation is chosen from a free-radical or anionic polymerization initiator, a raising or lowering of temperature, and light.

3. Use according to Claim 1 or 2, **characterized in that** the external stimulation is an anionic polymerization initiator.

4. Use according to Claim 3, **characterized in that** the initiator is water.

5. Use according to any one of Claims 1 to 4, in haircare cosmetics.

6. Use according to Claim 5, for styling, caring for, washing, dyeing, making up or shaping the hair.

7. Use according to Claim 5, for giving the hair body, volume or a bouffant effect.

8. Use according to Claim 5, for improving resistance to shampooing, in particular after dyeing.

9. Use according to any one of the preceding claims, **characterized in that** Z denotes a linear or branched C4 to C30 and preferably C6 to C28 divalent alkylene radical.

10. Use according to any one of the preceding claims, **characterized in that** the oxygenated heterocycle containing Z is an at least six-membered heterocycle.

11. Use according to any one of the preceding claims, **characterized in that** the compound (1) is chosen from those satisfying the following criteria:
(i) X = O, n = 0, R2 = S, Z = CH₂-CH(R)
(ii) X = O, n = 0, R2 = S, Z = CH₂-CH(R)-CH₂
(iii) X = S, n = 0, R2 = S, Z = CH₂-CH(R)
(iv) X = O, n = 0, R2 = O, Z = CH₂-C(R,R')-CH₂
(v) X = O, n = 0, R2 = O, Z = CH₂-CH₂-CH₂-CH₂ .

12. Use according to any one of the preceding claims, **characterized in that** the compound (1) is used, in a hair composition, in combination with at least one compound chosen from anionic, nonionic or amphoteric fixing polymers, conditioning agents, quaternized anionic or cationic direct dyes, oxidation dyes for the hair, reducing agents, surfactants and oxidizing agents.

13. Cosmetic composition comprising, in a cosmetically acceptable medium, a combination formed by (i) at least one compound chosen from anionic, nonionic or amphoteric fixing polymers, conditioning agents, quaternized anionic or cationic direct dyes, oxidation dyes for the hair, reducing agents, surfactants and oxidizing agents with (ii) at least one cyclic carbonate capable of polymerizing under the action of external stimulation, the cyclic carbonate being a compound of formula (1) 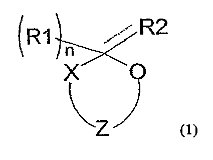 in which:
- X = O, S,
- n = 0,
- R2 = O, S,
- Z denotes a linear or branched C2 to C30 divalent alkylene radical, optionally interrupted with one or more heteroatoms, and optionally substituted, on the main chain or on the branching, with one or more C6 to C30 aryl, amino, halogen, carboxyl or thiol radicals,
Z being other than ethylene, propylene and isopropyl-2,2'-dimethylpropylene.

14. Composition according to Claim 13, **characterized in that** Z denotes a linear or branched C4 to C30 and preferably C6 to C28 divalent alkylene radical.

15. Composition according to Claim 13 or 14, **characterized in that** the concentration of compound (1) is between 0.001% and 50%, preferably between 0.01% and 30% and even more preferably between 0.1% and 20% by weight relative to the total weight of the composition.

16. Composition according to any one of Claims 13 to 15, **characterized in that** the concentration of compound chosen from anionic, nonionic or amphoteric fixing polymers or conditioning agents, anionic or cationic direct dyes or oxidation dyes for the hair, reducing agents, surfactants and oxidizing agents is between 0.001% and 50%, more preferably between 0.01% and 20% and even more preferably between 0.1% and 10%.

17. Composition according to any one of Claims 13 to 16, **characterized in that** the reducing agent is chosen from thioglycolic acid and its salts, thiolactic acid and its salts, cysteine, cysteamine, glyceryl thioglycolate, sulfites, bisulfites, sulfinates, ascorbic acid, erythorbic acid and glucose.

18. Composition according to any one of Claims 13 to 16, **characterized in that** the oxidizing agent is chosen from hydrogen peroxide, urea peroxide, alkali metal bromates, and persalts such as perborates, persulfates and periodates.

19. Composition according to any one of Claims 13 to 16, **characterized in that** the surfactant is chosen from anionic, cationic, nonionic and amphoteric surfactants.

20. Composition according to any one of Claims 13 to 16, **characterized in that** the oxidation dye is an oxidation base.

21. Composition according to Claim 20, **characterized in that** it contains at least one coupler.

22. Composition according to any one of Claims 13 to 16, **characterized in that** the direct dye is chosen from azo dyes, quinone dyes, azine dyes and triarylmethane dyes.

23. Composition according to any one of Claims 13 to 22, **characterized in that** it also contains one or more compounds chosen from pH regulators, polymerization initiators, such as metal salts, polymerization catalysts, for instance enzymes, solvents, for instance ethanol, antibacterial agents, nacreous agents, propellent gases, particles or nanoparticles, and pigments.

## Patentansprüche

1. Kosmetische Verwendung eines cyclischen Carbonats, das befähigt ist, unter der Einwirkung einer äußeren Stimulierung zu polymerisieren, in einem kosmetisch akzeptablen Medium, wobei das Carbonat eine Verbindung der Formel (I) ist: 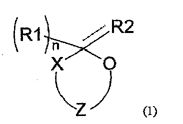 in der Formel:
- X = O, S,
- n = 0,
- R2 = O, S,
- Z bedeutet eine geradkettige oder verzweigte, zweiwertige Alkylengruppe mit 2 bis 30 Kohlenstoffatomen, die gegebenenfalls durch ein oder mehrere Heteroatome unterbrochen und gegebenenfalls an der Hauptkette oder an der Verzweigung mit einer oder mehreren Gruppen C₆₋₃₀-Aryl, Amino, Halogen, Carboxy, Thio substituiert ist,
wobei Z von Ethylen, Propylen und Isopropyl-2,2'-dimethylpropylen verschieden ist.

2. Verwendung nach Anspruch 1, **dadurch gekennzeichnet, dass** die äußere Stimulierung unter einem radikalischen oder anionischen Polymerisationsinitiator, einer Erhöhung oder einer Erniedrigung der Temperatur und Licht ausgewählt ist.

3. Verwendung nach Anspruch 1 oder 2, **dadurch gekennzeichnet, dass** es sich bei äußeren Stimulierung um einen anionischen Polymerisationsinitiator handelt.

4. Verwendung nach Anspruch 3, **dadurch gekennzeichnet, dass** der Initiator Wasser ist.

5. Verwendung nach einem der Ansprüche 1 bis 4 für die kosmetische Haarbehandlung.

6. Verwendung nach Anspruch 5 zum Frisieren, für die Pflege, für die Wäsche, zum Färben, zum Schminken oder für die Formgebung der Haare.

7. Verwendung nach Anspruch 5, um dem Haar Substanz, Volumen oder Stand zu geben.

8. Verwendung nach Anspruch 5, um insbesondere nach Färbungen die Beständigkeit gegenüber Haarwäschen zu verbessern.

9. Verwendung nach einem der vorhergehenden Ansprüche,
**dadurch gekennzeichnet, dass** Z eine lineare oder verzweigte, zweiwertige Alkylengruppe mit 4 bis 30 Kohlenstoffatomen und vorzugsweise 6 bis 28 Kohlenstoffatomen ist.

10. Verwendung nach einem der vorhergehenden Ansprüche,
**dadurch gekennzeichnet, dass** der sauerstoffhaltige Heterocyclus, der Z enthält, ein Heterocyclus ist, der mindestens 6-gliedrig ist.

11. Verwendung nach einem der vorhergehenden Ansprüche, **dadurch gekennzeichnet, dass** die Verbindung (I) unter den Verbindungen ausgewählt ist, die die nachfolgenden Kriterien erfüllen:
(i) X = O, n = 0, R2 = S, Z = CH₂-CH(R)
(ii) X = O, n = 0, R2 = S, Z = CH₂-CH(R)-CH₂
(iii) X = S, n = 0, R2 = S, Z = CH₂-CH(R)
(iv) X = O, n = 0, R2 = O, Z = CH₂-C(R, R')-CH₂
(v) X = O, n = 0, R2 = O, Z = CH₂-CH₂-CH₂-CH₂

12. Verwendung nach einem der vorhergehenden Ansprüche, **dadurch gekennzeichnet, dass** die Verbindung (I) in einer Zusammensetzung für die Haarbehandlung in Kombination mit mindestens einer Verbindung verwendet wird, die unter den anionischen, nichtionischen oder amphoteren fixierenden Polymeren, Konditioniermitteln, anionischen oder kationischen, quaternisierten Direktfarbstoffen, oxidativen Farbstoffen für die Haare, Reduktionsmitteln, grenzflächenaktiven Stoffen und Oxidationsmitteln ausgewählt ist.

13. Kosmetische Zusammensetzung, die in einem kosmetisch akzeptablen Medium eine Kombination enthält, die aus (i) mindestens einer Verbindung, die unter den anionischen, nichtionischen oder amphoteren fixierenden Polymeren, Konditioniermitteln, anionischen oder kationischen, quaternisierten Direktfarbstoffen, oxidativen Farbstoffen für die Haare, Reduktionsmitteln, grenzflächenaktiven Stoffen und Oxidationsmitteln ausgewählt ist, und (ii) mindestens einem cyclischen Carbonat gebildet ist, das befähigt ist, unter der Einwirkung einer äußeren Stimulierung zu polymerisieren, wobei das Carbonat eine Verbindung der Formel (I) ist: 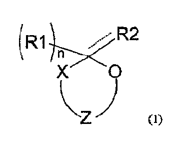 in der Formel:
- X = O, S,
- n = 0,
- R2 = O, S,
- Z bedeutet eine geradkettige oder verzweigte, zweiwertige Alkylengruppe mit 2 bis 30 Kohlenstoffatomen, die gegebenenfalls durch ein oder mehrere Heteroatome unterbrochen und gegebenenfalls an der Hauptkette oder an der Verzweigung mit einer oder mehreren Gruppen C₆₋₃₀-Aryl, Amino, Halogen, Carboxy, Thio substituiert ist,
wobei Z von Ethylen, Propylen und Isopropyl-2,2'-dimethylpropylen verschieden ist.

14. Zusammensetzung nach Anspruch 13, **dadurch gekennzeichnet, dass** Z eine lineare oder verzweigte, zweiwertige Alkylengruppe mit 4 bis 30 Kohlenstoffatomen und vorzugsweise 6 bis 28 Kohlenstoffatomen ist.

15. Zusammensetzung nach Anspruch 13 oder 14, **dadurch gekennzeichnet, dass** die Konzentration der Verbindung der Formel (I) im Bereich von 0,001 bis 50 Gew.-%, vorzugsweise im Bereich von 0,01 bis 30 Gew.-% und noch bevorzugter im Bereich von 0,1 bis 20 Gew.-%, bezogen auf das Gesamtgewicht der Zusammensetzung, liegt.

16. Zusammensetzung nach einem der Ansprüche 13 bis 15,
**dadurch gekennzeichnet, dass** die Konzentration der Verbindung, die unter den anionischen, nichtionischen oder amphoteren fixierenden Polymeren oder Konditioniermitteln, anionischen oder kationischen, quaternisierten Direktfarbstoffen oder oxidativen Farbstoffen für die Haare, Reduktionsmitteln, grenzflächenaktiven Stoffen und Oxidationsmitteln ausgewählt ist, im Bereich von 0,001 bis 50 Gew.-%, vorzugsweise 0,01 bis 20 Gew.-% und noch bevorzugter 0,1 bis 10 Gew.-%, bezogen auf das Gesamtgewicht der Zusammensetzung, liegt.

17. Zusammensetzung nach einem der Ansprüche 13 bis 16,
**dadurch gekennzeichnet, dass** das Reduktionsmittel unter Thioglycolsäure und ihren Salzen, Thiomilchsäure und ihren Salzen, Cystein, Cysteamin und Glycerinthioglycolat, Sulfiten, Bisulfiten, Sulfinaten, Ascorbinsäure, Erythorbinsäure und Glucose ausgewählt ist.

18. Zusammensetzung nach einem der Ansprüche 13 bis 16,
**dadurch gekennzeichnet, dass** das Oxidationsmittel unter Wasserstoffperoxid, Harnstoffperoxid, Alkalimetallbromaten, Salzen von Persäuren, wie Perboraten und Persulfaten, Perboraten ausgewählt ist.

19. Zusammensetzung nach einem der Ansprüche 13 bis 16,
**dadurch gekennzeichnet, dass** der grenzflächenaktive Stoff unter den anionischen, kationischen, nichtionischen oder amphoteren grenzflächenaktiven Stoffen ausgewählt ist.

20. Zusammensetzung nach einem der Ansprüche 13 bis 16,
**dadurch gekennzeichnet, dass** der Oxidationsfarbstoff eine Oxidationsbase ist.

21. Zusammensetzung nach Anspruch 20, **dadurch gekennzeichnet, dass** sie mindestens einen Kuppler enthält.

22. Zusammensetzung nach einem der Ansprüche 13 bis 16,
**dadurch gekennzeichnet, dass** der Direktfarbstoff unter den Azofarbstoffen, Chinon-Farbstoffen, Azin-Farbstoffen oder Triarylmethan-Farbstoffen ausgewählt ist.

23. Zusammensetzung nach einem der Ansprüche 13 bis 22,
**dadurch gekennzeichnet, dass** sie ferner eine oder mehrere Verbindungen enthält, die unter den pH-Reglern, Polymerisationsinitiatoren, wie Metallsalzen, Polymerisationskatalysatoren, wie Enzymen, Lösemitteln, wie Ethanol, antibakteriellen Wirkstoffen, Perlglanzstoffen, Treibmitteln, Partikeln oder Nanopartikeln oder Pigmenten ausgewählt sind.
